# EUROPEAN PATENT SPECIFICATION

(11) **EP 4 142 865 B1**
(45) Date of publication and mention of the grant of the patent: **28.10.2026**
(21) Application number: 21718586.7
(22) Date of filing: 16.04.2021
(51) Int. Cl.: A61N 1/365, A61N 1/368, A61N 1/36

(54) **HYPERTENSION THERAPY**
HYPERTONIE-THERAPIE
THÉRAPIE CONTRE L'HYPERTENSION

(30) Priority: 27.04.2020 EP 20171522
(43) Date of publication of application: 08.03.2023
(73) Proprietor: BIOTRONIK SE & Co. KG, 12359 Berlin (DE)
(72) Inventor: DÖRR, Thomas, 12437 Berlin (DE); WEISS, Ingo, 12435 Berlin (DE); BUSCH, Ulrich, 10318 Berlin (DE)
(74) Representative: Biotronik Corporate Services SE
(86) International application number: PCT/EP2021/059901
(87) International publication number: WO 2021/219403

(56) References cited:
- US-A1- 2004 215 266
- US-A1- 2010 174 333
- US-A1- 2016 193 470
- US-A1- 2016 220 824
- US-A1- 2016 310 733
- US-A1- 2019 299 003

## Description

The present invention relates to a device for treating hypertension by means of cardiac stimulation therapy.

A method for treating hypertension is for example disclosed in US 9,333,352 which describes methods for blood pressure lowering heart stimulation. The methods are based on making the timing inefficient so that the cardiac output decreases and, as a result, the blood pressure. As an example, US 9,333,352 teaches to deliver ventricular stimulation at the same time as atrial activity, or shortly after, or before. Particularly, US 9,333,353 discloses to deliver ventricular stimulation with a time delay of 0 to 50 ms after atrial activity.

US 2016/0220824 A1 describes a method that electrically stimulates a heart muscle to alter the ejection profile of the heart, to control the mechanical function of the heart and reduce the observed blood pressure of the patient. The therapy may be invoked by an implantable blood pressure sensor associated with a pacemaker like device.

US 2004/0215266 A1 teaches a pressure regulated atrio-ventricular delay for multi-site pacing. The hemodynamic operation of certain heart disease patient can be improved using pressure regulated atrio-ventricular delay. The pressure regulated atrio-ventricular delay involves recording atrial activation and ventricle activation, identifying a maximum diastolic pressure, identifying a declination pressure, calculating a percent decrease, and adjusting atrio-ventricular delay.

Thus, for patients with pacemakers, blood pressure can be lowered by physiologically inefficient stimulation in the atrium. However, for patients with intact AV transition this may mean unnecessary right ventricular stimulation.

Therefore, the problem to be solved by the present invention is to provide an implantable device that allows reducing the blood pressure permanently in combination with a cardiac stimulation that reduces the risk of the above-described unwanted effect of unnecessary right ventricular stimulation.

This problem is solved by an implantable device having the features of claim 1.

Preferred embodiments of these aspects of the present invention are stated in the corresponding sub claims and are described below.

Methods described hereinafter are not claimed as such, but are disclosed for better understanding the invention which is defined by the appended claims.

According to an embodiment, said delay is less than 100 ms, preferably less than 90 ms, preferably less than 80 ms, preferably less than 70 ms, preferably less than 60 ms, preferably less than 50 ms, preferably less than 40 ms, preferably less than 30 ms, preferably less than 20 ms, preferably less than 10 ms.

Furthermore, according to an embodiment, said delay is adjustable, wherein particularly the implantable device is configured to adjust the delay to be shorter than an intrinsic AV delay of the patient. For this, the stimulation unit can comprise a timer control circuit that enables the stimulation unit to perform said stimulation of the ventricle with an adjustable delay after detection of the atrial activity.

Furthermore, according to an embodiment, the implantable device comprises a ventricular sensing unit, and wherein the implantable device is configured to measure an intrinsic AV delay of the patient and to adjust said adjustable delay depending on the measured intrinsic AV delay, wherein the adjustable delay is adjusted to be shorter than said measured intrinsic AV delay.

The stimulation unit is configured to stimulate the ventricle irrespective of atrial activity for at least one cardiac cycle after the detection of an atrial activity or after the stimulation of an atrium in the absence of intrinsic AV conduction. This represents a safety function (VVI-backup-mode) of the implantable device in case no intrinsic AV delay has been measured. That function is particularly useful if no AV delay is used as pacing parameter.

In other words, the switching of the stimulation phases can be interval-controlled, i.e. after a number of heart cycles of atrial-controlled stimulation, the atrial-controlled stimulation is suspended for a number of heart cycles or replaced with stimulation without AV delay. Alternatively, the switching of the stimulation phases can be time-controlled, or it is time-and interval-controlled. Furthermore, the switching of the stimulation phases can be carried out depending on one or more parameters which can be assigned to the systemic blood pressure and which are recorded by the implantable device or another device.

Furthermore, according to an embodiment, the implantable device is configured to measure said parameter(s) or to receive said parameter(s) from another device.

Furthermore, according to an embodiment, the ventricle is the right ventricle or the ventricle is the left ventricle or the stimulation unit, is configured to stimulate the left and the right ventricle for at least one cardiac cycle with a delay after detection of an atrial activity or to not stimulate the left and the right ventricle for at least one cardiac cycle.

Furthermore, in case the right ventricle is stimulated, the implantable device can comprise a first electrode lead configured to be implanted in the right ventricle and to apply electrical stimulation to the right ventricle, and a second electrode lead configured to be implanted into the right atrium and configured to sense atrial activity of the right atrium.

Alternatively, in case the left ventricle is stimulated, the first electrode lead can be configured to be implanted in the left ventricle and to apply electrical stimulation to the left ventricle, while the second electrode lead can be configured as stated above (i.e. implanted in the right atrium).

Furthermore, in case both ventricles are stimulated, the implantable device can comprise a first electrode lead configured to be implanted in the right ventricle and to apply electrical stimulation to the right ventricle, and a second electrode lead configured to be implanted into the right atrium and configured to sense atrial activity of the right atrium, and a third electrode lead configured to be implanted in the left ventricle and to apply electrical stimulation to the left ventricle.

In all the cases stated above, alternatively, the atrial activity of the left atrium can be sensed instead of the atrial activity of the right atrium. For sensing the atrial activity of the left atrium, the respective electrode implanted in the left ventricle may comprise one or more additional electrode poles in the region of the left atrium to sense atrial activity of the left atrium, wherein the electrode in the right atrium can be omitted.

The present disclosure also relates to a non-claimed, exemplary method for lowering blood pressure by means of cardiac stimulation, wherein the method preferably uses an implantable device according to the present invention, wherein the ventricle is stimulated for at least one cardiac cycle with a predefined delay after detection of an atrial activity or after an atrial stimulation or subsequently not stimulated for at least one cardiac cycle.

Furthermore, according to the exemplary method, the ventricle is stimulated for at least one cardiac cycle with a predefined delay after detection of an atrial activity or with a predefined delay after the stimulation of an atrium and is subsequently not stimulated synchronously after atrial activity or an atrial stimulation for at least one cardiac cycle.

According to the exemplary method, the ventricle is stimulated irrespective of atrial activity for at least one cardiac cycle after the detection of an atrial activity or after the stimulation of an atrium in the absence of intrinsic AV conduction. This represents a safety function (VVI-backup-mode) of the implantable device in case no intrinsic AV delay has been measured. That function is particularly useful if no AV delay is used as pacing parameter.

According to the exemplary method, said adjustable delay is selected to be shorter than an intrinsic AV delay of the patient, particularly in case the patient does not suffer from Heart Failure with preserved Ejection Fraction (HF-PEF), or, in case the patient does suffer from HF-PEF, is selected to be shorter than an intrinsic AV delay which the patient would have on average based on e.g. at least age and sex if he/she did not suffer from HF-PEF.

According to the exemplary method, the cardiac stimulation is delivered to patients indicated with Heart Failure with preserved Ejection Fraction (HF-PEF).

Particularly, in case the patient has HF-PEF, the exemplary method is for permanently reducing end-diastolic pressure in humans suffering from HF-PEF, in particular.

In case the patient has HF-PEF, the cardiac stimulation of the ventricle based triggered by detection of atrial activity can also be applied to the His bundle or to the left bundle branch of the patient instead. Thus, regarding the above-described method and device the stimulation of the ventricle triggered by detection of atrial activity (e.g. using the delay being shorter than the intrinsic AV delay) can be substituted by a corresponding stimulation of the His bundle or the left bundle branch of the heart by the patient that is then triggered by detection of atrial activity, respectively.

In the following, embodiments, further features and advantages of the present invention shall be described with reference to the Figures which depict preferred embodiments of the present invention, wherein
- Figs. 1-4: show different embodiments of an implantable device according to the present invention; and
- Fig. 5: shows a flow chart, wherein the functions of the respective implantable device according to the present invention as well as of the exemplary method

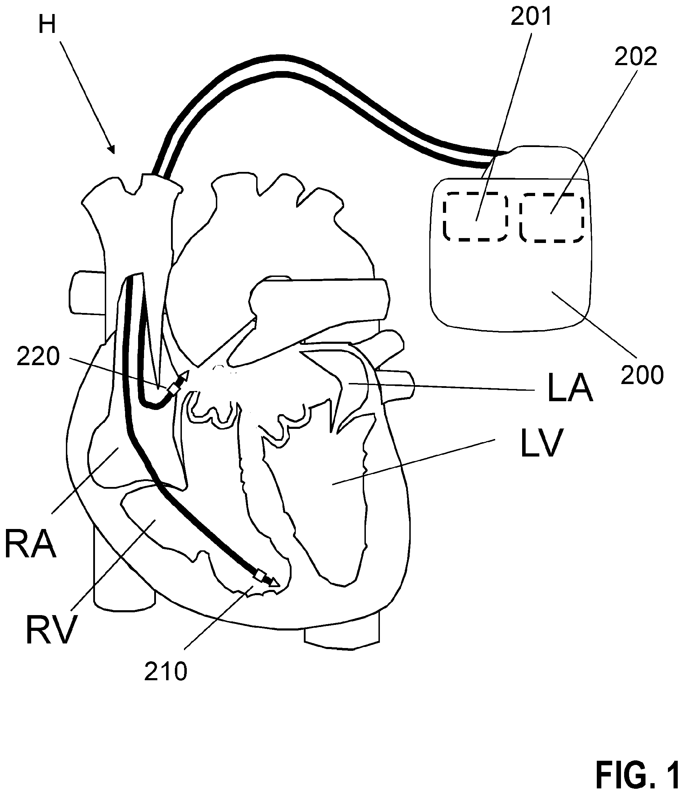

Fig. 1 shows an embodiment of an implantable device 200 for stimulating a heart H of a patient to lower blood pressure of a patient suffering from hypertension. Particularly, the method and device according to the present invention can be applied to a patient suffering from Heart Failure with preserved Ejection Fraction (HF-PEF) to permanently reduce end-diastolic pressure.

According to Fig. 1, the implantable device 200 comprises a stimulation unit 201 configured to stimulate a ventricle, particularly the right ventricle RV, and a sensing unit 202 for detecting atrial activity of the heart H, wherein the stimulation unit 201 is configured to stimulate the ventricle RV for at least one cardiac cycle with a predefined delay after detection of an atrial activity, and to subsequently stimulate the ventricle RV irrespective of atrial activity for at least one cardiac cycle or to not stimulate the ventricle RV for at least one cardiac cycle at all.

In case the right ventricle RV is stimulated as indicated in Fig. 1 for example, the implantable device 200 comprises a first electrode lead 210 configured to be implanted in the right ventricle RV and to apply electrical stimulation to the right ventricle RV generated by the stimulation unit 201, to which the first electrode lead 210 is electrically connected. Further, the implantable device 200 comprises a second electrode lead 220 configured to be implanted into the right atrium RA and configured to sense atrial activity of the right atrium RA. For this, the second electrode lead 220 is electrically connected to the sensing unit 202.

According to an embodiment of the present invention, stimulation unit 201 comprises a ventricular sensing unit for analyzing signals obtained via first electrode lead 210. Electrical signals measured via first electrode lead 210 are processed by the ventricular sensing unit for detection of activity of the right ventricle, for example.

Alternatively, the left ventricle LV can be stimulated instead. Furthermore, both ventricles RV and LV may also be stimulated.

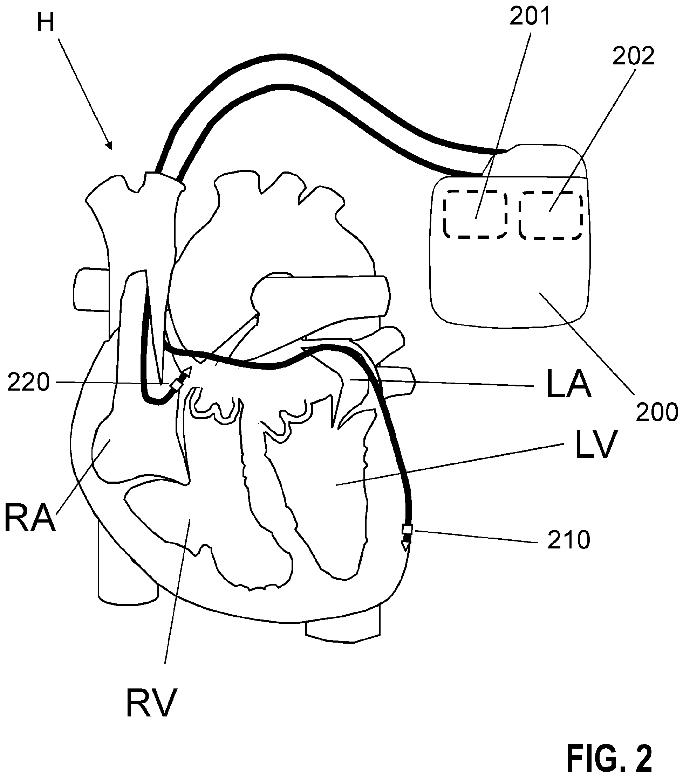

In case the left ventricle LV is stimulated as shown in Fig. 2, the first electrode lead 210 is configured to be implanted in the left ventricle LV and to apply electrical stimulation to the left ventricle LV, while the second electrode lead 220 can be configured as stated above (i.e. implanted in the right atrium).

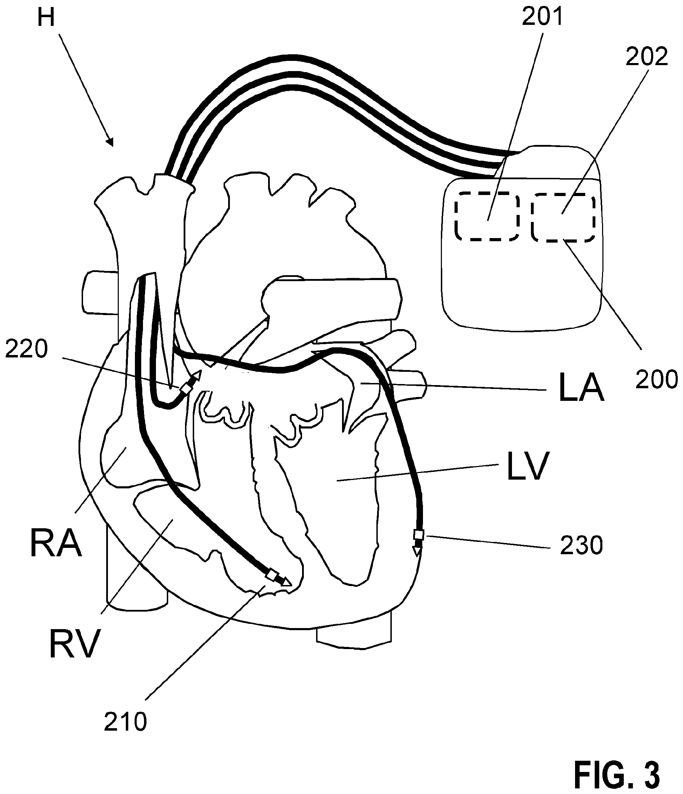

Furthermore, in case both ventricles are stimulated as shown in Fig 3, the implantable device 200 can comprise a first electrode lead 210 configured to be implanted in the right ventricle RV and to apply electrical stimulation to the right ventricle RV, and a second electrode lead 220 configured to be implanted into the right atrium RA and configured to sense atrial activity of the right atrium RA, and a third electrode lead 230 configured to be implanted in the left ventricle LV and to apply electrical stimulation to the left ventricle LV.

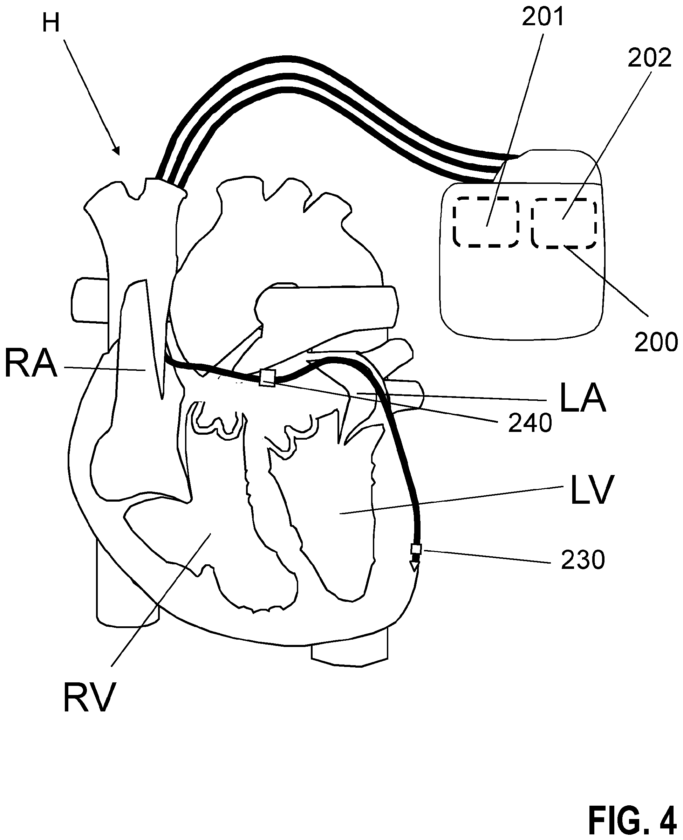

In all the cases stated above, instead of using the right atrium RA as shown in Figs. 1 to 3, the atrial activity of the left atrium LA may also be sensed instead as shown in Fig. 4. For sensing the atrial activity of the left atrium LA, the respective electrode 230 implanted in the left ventricle LV may comprise one or more additional electrode poles 240 in the region of the left atrium LA to sense atrial activity of the left atrium LA. Thus, the electrode in the right atrium RA can be omitted.

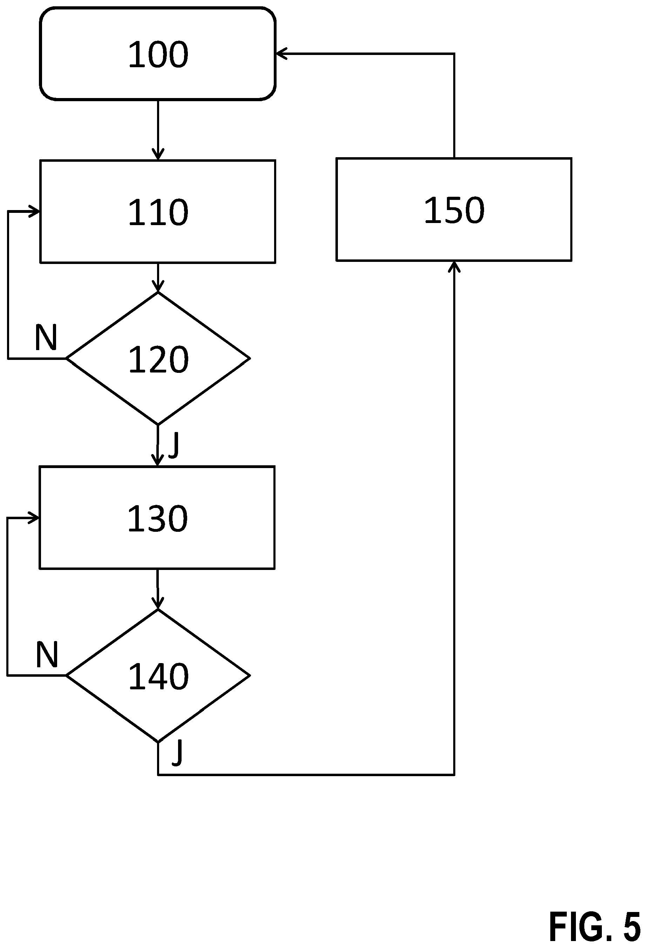

Fig. 5 shows a flow chart according to which an exemplary method for lowering blood pressure can be conducted or according to which functions of an implantable device 200 according to the present invention can be processed.

First, the blood pressure-lowering therapy is initiated 100 by setting atrial controlled stimulation with a shortened delay in the stimulation unit 201. Initially, a programmed value is preferably selected here for the delay, which is preset by the user so that this time is shorter than the intrinsic AV delay of the patient. Atrial signals are preferably sensed with the sensing unit 202, wherein an atrial signal from the right atriums RA (cf. Figs. 1 to 3) or from the left atrium LA may be used (cf. Fig. 4).

Subsequently, atrial-controlled ventricular stimulation takes place with this set delay 110, which causes the filling of the ventricle RV (and/or LV) to be terminated prematurely by the atrial contraction (reduced preload) and, as a result, a lower wall tension in the ventricle RV (and/or LV), which in turn causes a reduced inotropy and thus a reduction in the blood pressure for this heartbeat. This stimulation is repeated until a predefined number of ventricular stimulations 120, N has been performed.

When the number of ventricular intervals (heart cycles) is reached 120, J, the ventricular stimulation is switched off 130 and the ventricular rhythm is merely monitored or secured with a ventricular backup stimulation mode without atrial synchronization.

The suspension of the atrial controlled stimulation is performed with the aim of preventing blood pressure counter-regulation by the baroreceptors.

After a defined number of intrinsic heartbeats 140, J, the time duration of the current intrinsic AV delay of the patient is first measured and from this a shortened delay to be used for stimulations is determined 150. Then the atrial controlled stimulation 100, 110 by the stimulation unit 201 is reactivated with the newly determined delay.

The scope of the present invention is defined by the appended claims.

## Claims

1. An implantable device (200) for stimulating a heart (H) to lower blood pressure, comprising:
- a stimulation unit (201) configured to stimulate a ventricle, and
- a sensing unit (202) for detecting atrial activity of the heart (H),
wherein the stimulation (201) unit is configured to stimulate the ventricle (RV, LV) for at least one cardiac cycle with a predefined delay after detection of an atrial activity and to subsequently not stimulate the ventricle (RV, LV) with a predefined delay after the detection of an atrial activity for at least one cardiac cycle, wherein the implantable device is configured to suspend stimulation of the ventricle triggered by said detection of an atrial activity after a predefined number of heart cycles or after a predetermined time period for a number of heart cycles or for a predetermined time period,
**characterized in that**
the stimulation (201) unit is configured to stimulate the ventricle (RV, LV) irrespective of an AV delay for at least one cardiac cycle after the detection of an atrial activity in the absence of intrinsic AV conduction, wherein the implantable device (200) is configured to suspend the stimulation of the ventricle triggered by the detection of an atrial activity or triggered by an atrial stimulation depending on one or more parameters that can be assigned to the systemic blood pressure.

2. Implantable device (200) of claim 1, comprising an atrial stimulation unit (220) configured to stimulate an atrium,
wherein the stimulation (201) unit is configured to stimulate the ventricle (RV, LV) for at least one cardiac cycle with a predefined delay after detection of an atrial activity or with a predefined delay after the stimulation of an atrium and to subsequently not stimulate the ventricle (RV, LV) with a predefined delay after the detection of an atrial activity for at least one cardiac cycle, wherein the implantable device is configured to suspend stimulation of the ventricle triggered by said detection of an atrial activity or triggered by an atrial stimulation after a predefined number of heart cycles or after a predetermined time period for a number of heart cycles or for a predetermined time period,
wherein the stimulation (201) unit is configured to stimulate the ventricle (RV, LV) irrespective of an AV delay for at least one cardiac cycle after the detection of an atrial activity or after the stimulation of an atrium in the absence of intrinsic AV conduction.

3. The implantable device according to claim 1 or 2, wherein said delay is less than 100 ms, preferably less than 90 ms, preferably less than 80 ms, preferably less than 70 ms, preferably less than 60 ms, preferably less than 50 ms, preferably less than 40 ms, preferably less than 30 ms, preferably less than 20 ms, preferably less than 10 ms.

4. The implantable device according to one of the preceding claims, wherein said delay is adjustable, and wherein the implantable device (200) is configured to adjust the delay to be shorter than an intrinsic AV delay of the patient.

5. The implantable device according to one of the preceding claims, wherein the implantable device (200) comprises a ventricular sensing unit, and wherein the implantable device (200) is configured to measure an intrinsic AV delay of the patient and to adjust said adjustable delay depending on the measured intrinsic AV delay, wherein the adjustable delay is adjusted to be shorter than said measured intrinsic AV delay.

6. The implantable device according to claim 1, wherein the implantable device (200) is configured to measure the respective parameter or to receive the respective parameter from another device.

7. The implantable device according to one of the preceding claims, wherein the ventricle is the right ventricle (RV), or wherein the ventricle is the left ventricle (LV), or wherein the stimulation unit (201) is configured to stimulate the left and the right ventricle (RV, LV) for at least one cardiac cycle with said predefined delay after the detection of an atrial activity or after the stimulation of an atrium, and configured to subsequently not stimulate the left and the right ventricle (RV, LV) with a predefined delay after the detection of an atrial activity or with a predefined delay after the stimulation of an atrium for at least one cardiac cycle.

## Patentansprüche

1. Implantierbare Vorrichtung (200) zur Stimulation eines Herzens (H) zur Senkung des Blutdrucks, umfassend:
- eine Stimulationseinheit (201), die dazu eingerichtet ist, einen Ventrikel zu stimulieren, und
- eine Erfassungseinheit (202) zum Erfassen einer atrialen Aktivität des Herzens (H),
wobei die Stimulationseinheit (201) dazu eingerichtet ist, den Ventrikel (RV, LV) für mindestens einen Herzzyklus mit einer vordefinierten Verzögerung nach Erfassung einer atrialen Aktivität zu stimulieren und anschließend den Ventrikel (RV, LV) für mindestens einen Herzzyklus nicht mit einer vordefinierten Verzögerung nach der Erfassung einer atrialen Aktivität zu stimulieren, wobei die implantierbare Vorrichtung dazu eingerichtet ist, die durch die genannte Erfassung einer atrialen Aktivität ausgelöste Stimulation des Ventrikels nach einer vordefinierten Anzahl von Herzzyklen oder nach einem vorbestimmten Zeitraum für eine Anzahl von Herzzyklen oder für einen vorbestimmten Zeitraum auszusetzen,
**dadurch gekennzeichnet, dass**
die Stimulationseinheit (201) dazu eingerichtet ist, den Ventrikel (RV, LV) unabhängig von einer AV-Verzögerung für mindestens einen Herzzyklus nach der Erfassung einer atrialen Aktivität bei Fehlen einer intrinsischen AV-Überleitung zu stimulieren, wobei die implantierbare Vorrichtung (200) dazu eingerichtet ist, die durch die Erfassung einer atrialen Aktivität oder durch eine atriale Stimulation ausgelöste Stimulation des Ventrikels in Abhängigkeit von einem oder mehreren Parametern auszusetzen, die dem systemischen Blutdruck zugeordnet werden können.

2. Implantierbare Vorrichtung (200) nach Anspruch 1, umfassend eine atriale Stimulationseinheit (220), die dazu eingerichtet ist, ein Atrium zu stimulieren,
wobei die Stimulationseinheit (201) dazu eingerichtet ist, den Ventrikel (RV, LV) für mindestens einen Herzzyklus mit einer vordefinierten Verzögerung nach Erfassung einer atrialen Aktivität oder mit einer vordefinierten Verzögerung nach der Stimulation eines Atriums zu stimulieren und anschließend den Ventrikel (RV, LV) für mindestens einen Herzzyklus nicht mit einer vordefinierten Verzögerung nach der Erfassung einer atrialen Aktivität zu stimulieren, wobei die implantierbare Vorrichtung dazu eingerichtet ist, die durch die genannte Erfassung einer atrialen Aktivität oder durch eine atriale Stimulation ausgelöste Stimulation des Ventrikels nach einer vordefinierten Anzahl von Herzzyklen oder nach einem vorbestimmten Zeitraum für eine Anzahl von Herzzyklen oder für einen vorbestimmten Zeitraum auszusetzen,
wobei die Stimulationseinheit (201) dazu eingerichtet ist, den Ventrikel (RV, LV) unabhängig von einer AV-Verzögerung für mindestens einen Herzzyklus nach der Erfassung einer atrialen Aktivität oder nach der Stimulation eines Atriums bei Fehlen einer intrinsischen AV-Überleitung zu stimulieren.

3. Implantierbare Vorrichtung nach Anspruch 1 oder 2, wobei die genannte Verzögerung weniger als 100 ms beträgt, vorzugsweise weniger als 90 ms, vorzugsweise weniger als 80 ms, vorzugsweise weniger als 70 ms, vorzugsweise weniger als 60 ms, vorzugsweise weniger als 50 ms, vorzugsweise weniger als 40 ms, vorzugsweise weniger als 30 ms, vorzugsweise weniger als 20 ms, vorzugsweise weniger als 10 ms.

4. Implantierbare Vorrichtung nach einem der vorhergehenden Ansprüche, wobei die genannte Verzögerung einstellbar ist, und wobei die implantierbare Vorrichtung (200) dazu eingerichtet ist, die Verzögerung so einzustellen, dass sie kürzer als eine intrinsische AV-Verzögerung des Patienten ist.

5. Implantierbare Vorrichtung nach einem der vorhergehenden Ansprüche, wobei die implantierbare Vorrichtung (200) eine ventrikuläre Erfassungseinheit umfasst, und wobei die implantierbare Vorrichtung (200) dazu eingerichtet ist, eine intrinsische AV-Verzögerung des Patienten zu messen und die genannte einstellbare Verzögerung in Abhängigkeit von der gemessenen intrinsischen AV-Verzögerung einzustellen, wobei die einstellbare Verzögerung so eingestellt wird, dass sie kürzer als die genannte gemessene intrinsische AV-Verzögerung ist.

6. Implantierbare Vorrichtung nach Anspruch 1, wobei die implantierbare Vorrichtung (200) dazu eingerichtet ist, den jeweiligen Parameter zu messen oder den jeweiligen Parameter von einer anderen Vorrichtung zu empfangen.

7. Implantierbare Vorrichtung nach einem der vorhergehenden Ansprüche, wobei der Ventrikel der rechte Ventrikel (RV) ist, oder wobei der Ventrikel der linke Ventrikel (LV) ist, oder wobei die Stimulationseinheit (201) dazu eingerichtet ist, den linken und den rechten Ventrikel (RV, LV) für mindestens einen Herzzyklus mit der genannten vordefinierten Verzögerung nach der Erfassung einer atrialen Aktivität oder nach der Stimulation eines Atriums zu stimulieren, und dazu eingerichtet ist, anschließend den linken und den rechten Ventrikel (RV, LV) für mindestens einen Herzzyklus nicht mit einer vordefinierten Verzögerung nach der Erfassung einer atrialen Aktivität oder mit einer vordefinierten Verzögerung nach der Stimulation eines Atriums zu stimulieren.

## Revendications

1. Dispositif implantable (200) destiné à stimuler un cœur (H) pour diminuer la pression artérielle, comprenant :
- une unité de stimulation (201) configurée pour stimuler un ventricule, et
- une unité de détection (202) destinée à détecter l'activité atriale du cœur (H), dans lequel l'unité de stimulation (201) est configurée pour stimuler le ventricule (RV, LV) pendant au moins un cycle cardiaque avec un délai prédéfini après la détection d'une activité atriale et pour ensuite ne pas stimuler le ventricule (RV, LV) avec un délai prédéfini après la détection d'une activité atriale pendant au moins un cycle cardiaque, dans lequel le dispositif implantable est configuré pour suspendre la stimulation du ventricule déclenchée par ladite détection d'une activité atriale après un nombre prédéfini de cycles cardiaques ou après une période prédéterminée pour un nombre de cycles cardiaques ou pour une période prédéterminée,
**caractérisé en ce que**
l'unité de stimulation (201) est configurée pour stimuler le ventricule (RV, LV) indépendamment d'un délai AV pendant au moins un cycle cardiaque après la détection d'une activité atriale en l'absence d'une conduction AV intrinsèque, dans lequel le dispositif implantable (200) est configuré pour suspendre la stimulation du ventricule déclenchée par la détection d'une activité atriale ou déclenchée par une stimulation atriale en fonction d'un ou de plusieurs paramètres qui peuvent être attribués à la pression artérielle systémique.

2. Dispositif implantable (200) selon la revendication 1, comprenant une unité de stimulation atriale (220) configurée pour stimuler un atrium,
dans lequel l'unité de stimulation (201) est configurée pour stimuler le ventricule (RV, LV) pendant au moins un cycle cardiaque avec un délai prédéfini après la détection d'une activité atriale ou avec un délai prédéfini après la stimulation d'un atrium et pour ensuite ne pas stimuler le ventricule (RV, LV) avec un délai prédéfini après la détection d'une activité atriale pendant au moins un cycle cardiaque, dans lequel le dispositif implantable est configuré pour suspendre la stimulation du ventricule déclenchée par ladite détection d'une activité atriale ou déclenchée par une stimulation atriale après un nombre prédéfini de cycles cardiaques ou après une période prédéterminée pour un nombre de cycles cardiaques ou pendant une période prédéterminée,
dans lequel l'unité de stimulation (201) est configurée pour stimuler le ventricule (RV, LV) indépendamment d'un délai AV pendant au moins un cycle cardiaque après la détection d'une activité atriale ou après la stimulation d'un atrium en l'absence d'une conduction AV intrinsèque.

3. Dispositif implantable selon la revendication 1 ou 2, dans lequel ledit délai est de moins de 100 ms, de préférence de moins de 90 ms, de préférence de moins de 80 ms, de préférence de moins de 70 ms, de préférence de moins de 60 ms, de préférence de moins de 50 ms, de préférence de moins de 40 ms, de préférence de moins de 30 ms, de préférence de moins de 20 ms, de préférence de moins de 10 ms.

4. Dispositif implantable selon l'une des revendications précédentes, dans lequel ledit délai est ajustable, et dans lequel le dispositif implantable (200) est configuré pour ajuster le délai pour qu'il soit plus court qu'un délai AV intrinsèque du patient.

5. Dispositif implantable selon l'une des revendications précédentes, dans lequel le dispositif implantable (200) comprend une unité de détection ventriculaire, et dans lequel le dispositif implantable (200) est configuré pour mesurer un délai AV intrinsèque du patient et pour ajuster ledit délai ajustable en fonction du délai AV intrinsèque mesuré, dans lequel le délai ajustable est ajusté pour être plus court que le délai AV intrinsèque mesuré.

6. Dispositif implantable selon la revendication 1, dans lequel le dispositif implantable (200) est configuré pour mesurer le paramètre respectif ou pour recevoir le paramètre respectif depuis un autre dispositif.

7. Dispositif implantable selon l'une des revendications précédentes, dans lequel le ventricule est le ventricule droit (RV), ou dans lequel le ventricule est le ventricule gauche (LV), ou dans lequel l'unité de stimulation (201) est configurée pour stimuler le ventricule gauche et le ventricule droit (RV, LV) pendant au moins un cycle cardiaque avec ledit délai prédéfini après la détection d'une activité atriale ou après la stimulation d'un atrium, et configurée pour ensuite ne pas stimuler le ventricule gauche et le ventricule droit (RV, LV) avec un délai prédéfini après la détection d'une activité atriale ou avec un délai prédéfini après la stimulation d'un atrium pendant au moins un cycle cardiaque.
